# EUROPEAN PATENT SPECIFICATION

(11) **EP 1 993 619 B1**
(45) Date of publication and mention of the grant of the patent: **17.04.2013**
(21) Application number: 07758208.8
(22) Date of filing: 09.03.2007
(51) Int. Cl.: A61K 33/30, A61K 9/00, A61P 27/02, A61K 45/06, A61L 12/08, A61L 12/14, C11D 3/04, C11D 3/06, C11D 3/20, C11D 3/22, C11D 3/48

(54) **PHARMACEUTICAL FORMULATIONS COMPRISING POLYANIONIC MATERIALS AND ZINC-BASED PRESERVATIVES**
PHARMAZEUTISCHE FORMULIERUNG MIT POYLANIONISCHEN MATERIALIEN UND KONSERVIERUNGSMITTELN AUF ZINKBASIS
FORMULATIONS PHARMACEUTIQUES COMPRENANT DES SUBSTANCES POLYANIONIQUES ET DES PRESERVATIFS A BASE DE ZINC

(30) Priority: 10.03.2006 US 373571
(43) Date of publication of application: 26.11.2008
(73) Proprietor: BAUSCH & LOMB INCORPORATED, Rochester, NY 14604-2701 (US)
(72) Inventor: DOBIE, Alyce, K., Williamson, NY 14589 (US); KLEIBER, Tammy, J., Rochester, NY 14616 (US); LAVOIE, Paul, T., Pittsford, NY 14534 (US); XIA, Erning, Penfield, NY 14526 (US)
(74) Representative: Glas, Holger
(86) International application number: PCT/US2007/063634
(87) International publication number: WO 2007/106723

(56) References cited:
- EP-A1- 0 436 284
- WO-A-95/05804
- WO-A-98/32435
- WO-A-2005/097067
- WO-A-2006/068899
- WO-A1-98/10773
- US-A- 4 138 477
- US-A- 4 631 072
- DATABASE WPI Week 200377 Derwent Publications Ltd., London, GB; AN 2003-818778 XP002448635 & JP 2003 160464 A (DENKI KAGAKU KOGYO KK) 3 June 2003 (2003-06-03)
- DATABASE WPI Week 200377 Derwent Publications Ltd., London, GB; AN 2003-818284 XP002448636 & JP 2003 146892 A (ROHTO SEIYAKU KK) 21 May 2003 (2003-05-21)

## Description

### BACKGROUND OF THE INVENTION

The present invention relates to pharmaceutical formulations comprising polyanionic materials and zinc-based preservatives. In particular, the present invention relates to ophthalmic solutions that provide improved safety and/or comfort to the users.

Pharmaceutical formulations are commonly provided in multi-use bottles. Formulations, such as ophthalmic solutions, find uses in many ophthalmic applications. These solutions are often instilled directly into the eye one or more times a day to either deliver medications or to relieve symptoms of eye conditions, such as dry eye or inflammation of the superficial tissues of the eye accompanying various allergic reactions (such as hay fever allergies and the like, irritation of the eye due to foreign bodies, or eye fatigue). Other ophthalmic solutions are employed in the field of contact-lens care. Contact lens solutions are utilized to soak, disinfect, clean, and wet contact lenses. These solutions are not instilled directly in the eye from the bottle, but do subsequently come into contact with the eye when the lenses are inserted.

Ophthalmic solutions are provided sterile, but once opened, are susceptible to microbial contamination. In the case of multi-use solutions, the formulations contain at least a preservative designed to kill microorganisms that come in contact with the solution, protecting the patient from infection due to a contaminated ophthalmic solution during the prescribed usage.

Traditionally, preservatives for ophthalmic solutions fall into one or two categories: alcohols and amines or ammonium-containing compounds. Typical alcohol-based anti-microbial agents include benzyl alcohol, phenethyl alcohol, and chlorbutanol. These alcohols have limited solubility in aqueous solutions and tend not to be stable preservatives due to being susceptible to oxidation, evaporation, and interaction with the plastic bottle. More commonly, organic amines and ammonium-containing compounds are utilized as anti-microbial agents in ophthalmic solutions. Representative compounds in this category include benzalkonium chloride ("BAK"), chlorhexidine, polymeric biguanide (such as polyhexamethylene biguanide or "PHMB"). It is believed that the electrophilicity of the nitrogen-containing moieties of these compounds promotes their interaction with the negatively charged cell membranes of the microorganisms, thus severely impacting their survival.

Although amines and ammonium-containing compounds have acceptable anti-microbial activity, and are used commercially to preserve ophthalmic solutions, there are significant disadvantages associated with these compounds. In particular, these compounds used at higher doses can be toxic to the sensitive tissues of the eye. For example, BAK-containing ophthalmic solutions are known to cause eye irritation in patients. Polymeric amines and ammonium-containing compounds are less toxic than BAK but still can cause irritation responses in some other patients. Chlorhexidine, on the other hand, has proven to be more biocompatible than the other amines and ammonium-containing anti-microbial agents and, therefore, non-irritating at the levels typically used.

However, the mildness of chlorhexidine to the ocular environment is offset by the fact that chlorhexidine is a relatively weak preservative.

WO 98/32435 A1 refers to a composition that is used as an eye treatment containing reduced glutathione, vitamin A and vitamin E, as well as one or more of zinc sulfate, boric acid and potassium as buffering agents. The composition also may contain a lubricant and a preservative. The composition is a sterile isotonic solution. The composition is used in a method of treating eyes for the alleviation of irritations and/or dryness, as well as for the prevention and treatment of cataracts.

WO 95/05804 A1 refers to ophthalmic pharmaceutical vehicles which increase in viscosity upon instillation in the eye. Ophthalmic compositions of the vehicle and a pharmaceutically active drug are also disclosed.

US 4,631,072 A relates to a mixture for the care and cleaning of contact lenses, containing water, an abrasive based on one or several metal oxides, and a suspension aid based on swellable compounds. The abrasive is zinc oxide and/or tin oxide having a grain size smaller than 10 µm and a suspension aid based on swellable compounds, such as polyvinyl alcohol, cellulose derivatives, guar gum, as well as optionally buffers for adjusting the pH value to between 5 and 8 and/or sodium chloride, the ratio of abrasive to water in the mixture ranging between 1:99 and 90:10, and the grain size of the abrasive being preferably in a range of 0.5 µm.

JP 2003/160464 A relates to a stabilization composition of aqueous solution of hyaluronic acid and/or its salts used in pharmaceuticals, such as knee-osteoarthritis therapeutic agent, and cosmetics.

EP 0 436 284 A1 relates to a stable and clear aqueous solution of zinc salt useful as a mouthrinse and a gel dentifrice. In a preferred embodiment the aqueous solution comprises zinc chloride codissolved with a complexing agent of sodium gluconate, in a naturally derived anionic polymer of sodium carboxymethylcellulose.

US 4,138,477 A relates to a composition to prevent and control mouth odor, which is also effective in preventing calculus, plaque, caries and periodontal disease containing as the essential agent, a zinc-polymer combination formed by the reaction or interaction of a zinc compound with an anionic polymer containing carboxylic, sulfonic and/or phosphonic acid radicals.

WO 2005/097067 A1 relates to a composition comprising a preservative-effective amount of a zinc compound and less than a preservative-effective amount of a primary preservative agent-preferably no primary preservative agent.

WO 2006/068899 A2 relates to formulations containing Aloe Vera. These formulations are useful as eye drops that provide high patient compliance as well as carriers for pharmaceuticals such as steroids and anti-allergy ophthalmic drugs.

WO 98/10773 A1 relates to pharmaceutical compositions of antimicrobial effect as well as a process for the preparation thereof. The pharmaceutical compositions of the invention comprise zinc or cobalt hyaluronate associate (complex) as active ingredient in admixture with a carrier and/or other additives commonly used in the pharmaceutical industry.

Therefore, there is a continued need to provide improved pharmaceutical formulations that are effective in killing microorganisms or in inhibiting their growth and that provide improved safety and/or comfort to the users. It is also very desirable to provide improved ophthalmic solutions having such advantages.

### BRIEF SUMMARY OF THE INVENTION

In general, the present invention provides improved pharmaceutical formulations that are effective in adversely affecting the viability of microorganisms or in inhibiting their growth and that provide better safety and/or comfort to the users.

In general, a pharmaceutical formulation of the present invention comprises at least a polyanionic material and at least a zinc-based compound.

In one aspect, such a pharmaceutical formulation is an ophthalmic solution, which provides less irritation to ocular tissues and more lubricity to ocular surfaces than prior-art solutions.

In another aspect, said at least a zinc-based compound is present in an effective amount to inhibit or prevent the survival of microorganisms.

In still another aspect, representatives of such microorganisms comprise *Staphylococcus aureus, Pseudomonas aeruginosa, Eschrechia coli, Candida albicans,* and *Aspergillus niger.*

In yet another aspect, a pharmaceutical formulation of the present invention is free of cationic organic nitrogen-containing compounds.

In a further aspect, the present invention provides a method for making a pharmaceutical formulation. The method comprises providing at least a polyanionic material and at least a zinc-based compound in the pharmaceutical formulation.

In still another aspect, the present invention provides a method for providing safety, or comfort, or both to users of pharmaceutical formulation. The method comprises adding at least a polyanionic material and at least a zinc-based compound to the pharmaceutical formulation.

In yet another aspect, the present invention provide an ophthalmic solution for use in a method for treating or preventing a condition of an eye that manifests irritation or inflammation. The method comprises topically administering to the eye an effective amount of an ophthalmic solution that comprises at least a polyanionic material and at least a soluble zinc compound to relieve such irritation or inflammation.

In a further aspect, the present invention provides a method for treating an ophthalmic device. The method comprises contacting the ophthalmic device with an ophthalmic solution comprising at least a polyanionic material and at least a zinc-based compound.

In still a further aspect, the ophthalmic device is a contact lens.

One aspect of the present invention refers to an ophthalmic pharmaceutical formulation consisting of purified water and:
(a) 0.06 wt.-% sodium borate; 0.7 wt.-% boric acid; 1wt.-% glycerin, 0.25 wt.-% sodium alginate; 0.025 wt.-% zinc chloride; and optionally 0.01 wt.-% magnesium chloride; or
(b) 0.115 wt.-% sodium borate; 0.5 wt.-% boric acid; 1 wt.-% glycerin, 0.25 wt.-% sodium alginate; 0.01 wt.-% zinc chloride; and 0.01 wt.-% magnesium chloride; or
(c) 0.014 wt.-% sodium borate; 0.5 wt.-% boric acid; 1 wt.-% glycerin, 0.25 wt.-% or 0.5 wt.-% sodium hyaluronate; 0.02 wt.-% zinc chloride; and 0.01 wt.-% magnesium chloride; or
(d) 0.014 wt.-% sodium borate; 0.5 wt.-% boric acid; 1 wt.-% glycerin, 0.25 wt.-% or 0.5 wt.-% sodium hyaluronate; 0.02 wt.-% zinc chloride; and 0.02 wt.-% magnesium chloride; or
(e) 0.014 wt.-% sodium borate; 0.5 wt.-% boric acid; 1 wt.-% glycerin, 0.25 wt.-% sodium hyaluronate; 0.025 wt.-% zinc chloride; and 0.01 wt.-% magnesium chloride; or
(f) 0.014 wt.-% sodium borate; 0.5 wt.-% boric acid; 1 wt.-% glycerin, 0.25 wt.-% sodium hyaluronate; and 0.01 wt.-% or 0.02 wt.-% or 0.03 wt.-% zinc chloride;
wherein all wt.-% are based on the total weight of the formulation.

A further aspect of the present invention refers to an ophthalmic solution for use in treating of or preventing of a condition of an eye that manifests irritation or inflammation, the solution comprising an effective amount of the ophthalmic pharmaceutical formulation.

Another aspect of the present invention refers to a method for treating an ophthalmic device, the method comprising contacting the ophthalmic device with the ophthalmic pharmaceutical formulation.

Other features and advantages of the present invention will become apparent from the following detailed description and claims.

### DETAILED DESCRIPTION

In general, the present invention provides improved pharmaceutical formulations that are effective in adversely affecting the viability of microorganisms or in inhibiting their growth and that provide improved safety and/or comfort to the users, methods of making, and methods of using such formulations. Within the scope of the present invention, the microorganisms that are adversely affected by a formulation of the present invention include bacteria, yeasts, and/or molds.

In one aspect, pharmaceutical formulations of the present invention can kill or adversely affect the survival or propagation of such microorganisms. In one embodiment, representatives of such microorganisms comprise *Staphylococcus aureus* (or *S. aureus*), *Pseudomonas aeruginosa* (or *P. aeruginosa*), *Eschrechia coli* (or *E. coli*), *Candida albicans* (or *C*. *albicans*), and *Aspergillus niger* (or *A. niger*).

In another aspect, a pharmaceutical formulation of the present invention comprises at least a polyanionic material and at least a zinc-based compound. The term "polyanionic material," as used herein, means a material a molecule of which comprises a plurality of negatively charged moieties and carries a net negative charge. In one embodiment, the pharmaceutical formulation comprises an ophthalmic solution.

In still another aspect, an ophthalmic solution of the present invention provides less irritation to ocular tissues and more lubricity to ocular surfaces than prior-art solutions.

In yet another aspect, said at least a zinc-based compound is present in an effective amount to inhibit or prevent the survival of microorganisms. In one embodiment, the effectiveness of the solution is determined according to a testing procedure disclosed below.

In one embodiment, said at least a zinc-based compound is zinc chloride.

In another embodiment, said at least a soluble zinc compound is present In an amount effective to adversely affect the viability of microorganisms or inhibit their growth. In still another embodiment, said amount is effective to reduce the concentration of viable bacteria, recovered per milliliter of the solution, at the fourteenth day after challenge, by not less than 3 logs, and after a rechallenge at the fourteenth day, said amount is also effective to reduce the concentration of viable bacteria, recovered per milliliter of the solution, at the twenty-eighth day, by not less than 3 logs. In addition, said amount is effective to keep the concentration of viable yeasts and molds, recovered per milliliter of the solution, at or below the initial concentration (within an experimental uncertainty of ± 0.5 log) at the fourteenth day, and after a rechallenge at the fourteenth day, said amount is also effective to keep the concentration of viable yeasts and molds, recovered per milliliter of the solution, at or below the Initial concentration (within an experimental uncertainty of ± 0.5 log) at the twenty-eighth day.

In still another embodiment, the amount of the soluble zinc compound is in the range from about 0.0001 to about 5 percent by weight of the solution. Alternatively, the amount of the soluble zinc compound is in the range from about 0.001 to about 2 percent, or from about 0.001 to about 1 percent, or from about 0.01 to about 0.7 percent, or from about 0.01 to about 0.5 percent, or from about 0.01 to about 0.2 percent, or from about 0.01 to about 0.1 percent, or from about 0.01 to about 0.05 percent by weight of the solution.

In another aspect, the polyanionic material comprises an anionic derivative of a polysaccharide.

In still another aspect, the polyanionic material is selected from physiologically acceptable salts of alginic acid, hyaluronic acid;

In one embodiment, the amount of the polyanionic material in an ophthalmic solution of the present invention is in the range from about 0.01 to about 10 percent by weight of the solution. Alternatively, the amount of the polyanionic material is in the range from about 0.01 to about 5 percent, or from about 0.02 to about 2 percent, or from about 0.05 to about 1 percent, or from about 0.1 to about 0.5 percent by weight of the solution. In another embodiment, the polyanionic material Is present in the solution in an amount sufficient to provide lubrication to an ocular surface, such as the corneal or the conjunctiva.

In yet another aspect, an ophthalmic solution of the present invention is free of cationic organic nitrogen-containing compounds, such as cationic organic nitrogen-containing small molecules or polymers.

An ophthalmic solution of the present invention can further comprise one or more other ingredients, such as therapeutic agents that target specific eye conditions, buffers, tonicity adjusting agents, surfactants, viscosity adjusting agents, or other components.

For example, an ophthalmic solution of the present invention can comprise a therapeutic agent such as anti-inflammatory agents, antibiotics, immunosuppressive agents, antiviral agents, antifungal agents, antiprotozoal agents, combinations thereof, or mixtures thereof. Non-limiting examples of anti-inflammatory agents include glucocorticosteroids (e.g., for short-term treatment) and non-steroidal anti-inflammatory drugs ("NSAIDs").

Non-limiting examples of the glucocorticosteroids are: 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortarnate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, their physiologically acceptable salts, derivatives thereof, combinations thereof, and mixtures thereof. In one embodiment, the therapeutic agent is selected from the group consisting of difluprednate, loteprednol etabonate, prednisolone, combinations thereof, and mixtures thereof.

Non-limiting examples of the NSAIDs are: aminoarylcarboxylic acid derivatives (e.g., enfenamic acid, etofenamate, flufenamic acid, isonixin, meclofenamic acid, mefenamic acid, niflumic acid, talniflumate, terofenamate, tolfenamic acid), arylacetic acid derivatives (e.g., aceclofenac, acemetacin, alclofenac, amfenac, amtolmetin guacil, bromfenac, bufexamac, cinmetacin, clopirac, diclofenac sodium, etodolac, felbinac, fenclozic acid, fentiazac, glucametacin, ibufenac, indomethacin, isofezolac, isoxepac, lonazolac, metiazinic acid, mofezolac, oxametacine, pirazolac, proglumetacin, sulindac, tiaramide, tolmetin, tropesin, zomepirac), arylbutyrric acid derivatives (e.g., bumadizon, butibufen, fenbufen, xenbucin), arylcarboxylic acids (e.g., clidanac, ketorolac, tinoridine), arylpropionic acid derivatives (e.g., alminoprofen, benoxaprofen, bermoprofen, bucloxic acid, carprofen, fenoprofen, flunoxaprofen, flurbiprofen, ibuprofen, ibuproxam, indoprofen, ketoprofen, loxoprofen, naproxen, oxaprozin, piketoprolen, pirprofen, pranoprofen, protizinic acid, suprofen, tiaprofenic acid, ximoprofen, zaltoprofen), pyrazoles (e.g., difenamizole, epirizole), pyrazolones (e.g., apazone, benzpiperylon, feprazone, mofebutazone, morazone, oxyphenbutazone, phenylbutazone, pipebuzone, propyphenazone, ramifenazone, suxibuzone, thiazolinobutazone), salicylic acid derivatives (e.g., acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide o-acetic acid, salicylsulfuric acid, salsalate, sulfasalazine), thiazinecarboxamides (e.g., ampiroxicam, droxicam, isoxicam, lornoxicam, piroxicam, tenoxicam), ε-acetamidocaproic acid, S-(5'-adenosyl)-L-methionine, 3-amino-4-hydroxybutyric acid, amixetrine, bendazac, benzydamine, α-bisabolol, bucolome, difenpiramide, ditazol, emorfazone, fepradinol, guaiazulene, nabumetone, nimesulide, oxaceprol, paranyline, perisoxal, proquazone, superoxide dismutase, tenidap, zileuton, their physiologically acceptable salts, combinations thereof, and mixtures thereof.

Non-limiting examples of antibiotics include doxorubicin; aminoglycosides (e.g., amikacin, apramycin, arbekacin, bambermycins, butirosin, dibekacin, dihydrostreptomycin, fortimicin(s), gentamicin, isepamicin, kanamycin, micronomicin, neomycin, neomycin undecylenate, netilmicin, paromomycin, ribostamycin, sisomicin, spectinomycin, streptomycin, tobramycin, trospectomycin), amphenicols (e.g., azidamfenicol, chloramphenicol, florfenicol, thiamphenicol), ansamycins (e.g., rifamide, rifampin, rifamycin SV, rifapentine, rifaximin), β-lactams (e.g., carbacephems (e.g., loracarbef)), carbapenems (e.g., biapenem, imipenem, meropenem, panipenem), cephalosporins (e.g., cefaclor, cefadroxil, cefamandole, cefatrizine, cefazedone, cefazolin, cefcapene pivoxil, cefclidin, cefdinir, cefditoren, cefepime, cefetamet, cefixime, cefinenoxime, cefodizime, cefonicid, cefoperazone, ceforamide, cefotaxime, cefotiam, cefozopran, cefpimizole, cefpiramide, cefpirome, cefpodoxime proxetil, cefprozil, cefroxadine, cefsulodin, ceftazidime, cefteram, ceftezole, ceftibuten, ceftizoxime, ceftriaxone, cefuroxime, cefuzonam, cephacetrile sodium, cephalexin, cephaloglycin, cephaloridine, cephalosporin, cephalothin, cephapirin sodium, cephradine, pivcefalexin), cephamycins (e.g., cefbuperazone, cefinetazole, cefininox, cefotetan, cefoxitin), monobactams (e.g., aztreonam, carumonam, tigemonam), oxacephems, flomoxef, moxalactam), penicillins (e.g., amdinocillin, amdinocillin pivoxil, amoxicillin, ampicillin, apalcillin, aspoxicillin, azidocillin, azlocillin, bacampicillin, benzylpenicillinic acid, benzylpenicillin sodium, carbenicillin, carindacillin, clometocillin, cloxacillin, cyclacillin, dicloxacillin, epicillin, fenbenicillin, floxacillin, hetacillin, lenampicillin, metampicillin, methicillin sodium, mezlocillin, nafcillin sodium, oxacillin, penamecillin, penethamate hydriodide, penicillin G benethamine, penicillin G benzathine, penicillin G benzhydrylamine, penicillin G calcium, penicillin G hydrabamine, penicillin G potassium, penicillin G procaine, penicillin N, penicillin O, penicillin V, penicillin V benzathine, penicillin V hydrabamine, penimepicycline, phenethicillin potassium, piperacillin, pivampicillin, propicillin, quinacillin, sulbenicillin, sultamicillin, talampicillin, temocillin, ticarcillin), lincosamides (e.g., clindamycin, lincomycin), macrolides (e.g., azithromycin, carbomycin, clarithromycin, dirithromycin, erythromycin, erythromycin acistrate, erythromycin estolate, erythromycin glucoheptonate, erythromycin lactobionate, erythromycin propionate, erythromycin stearate, josamycin, leucomycins, midecamycins, miokamycin, oleandomycin, primycin, rokitamycin, rosaramicin, roxithromycin, spiramycin, troleandomycin), polypeptides (e.g., amphomycin, bacitracin, capreomycin, colistin, enduracidin, enviomycin, fusafungine, gramicidin S, gramicidin(s), mikamycin, polymyxin, pristinamycin, ristocetin, teicoplanin, thiostrepton, tuberactinomycin, tyrocidine, tyrothricin, vancomycin, viomycin, virginiamycin, zinc bacitracin), tetracyclines (e.g., apicycline, chlortetracycline, clomocycline, demeclocycline, doxycycline, guamecycline, lymecycline, meclocycline, methacycline, minocycline, oxytetracycline, penimepicycline, pipacycline, rolitetracycline, sancycline, tetracycline), and others (e.g., cycloserine, mupirocin, tuberin).

Other examples of antibiotics are the synthetic antibacterials, such as 2,4-diaminopyrimidines (e.g., brodimoprim, tetroxoprim, trimethoprim), nitrofurans (e.g., furaltadone, furazolium chloride, nifuradene, nifuratel, nifurfoline, nifurpirinol, nifurprazine, nifurtoinol, nitrofurantoin), quinolones and analogs (e.g., cinoxacin, ciprofloxacin, clinafloxacin, difloxacin, enoxacin, fleroxacin, flumequine, grepafloxacin, lomefloxacin, miloxacin, nadifloxacin, nalidixic acid, norfloxacin, ofloxacin, oxolinic acid, pazufloxacin, pefloxacin, pipemidic acid, piromidic acid, rosoxacin, rufloxacin, sparfloxacin, temafloxacin, tosufloxacin, trovafloxacin), sulfonamides (e.g., acetyl sulfamethoxypyrazine, benzylsulfamide, chloramine-B, chloramine-T, dichloramine T, n²-formylsulfisomidine, n⁴-β-D-glucosylsulfanilamide, mafenide, 4'-(methylsulfamoyl)sulfanilanilide, noprylsulfamide, phthalylsulfacetamide, phthalylsulfathiazole, salazosulfadimidine, succinylsulfathiazole, sulfabenzamide, sulfacetamide, sulfachlorpyridazine, sulfachrysoidine, sulfacytine, sulfadiazine, sulfadicramide, sulfadimethoxine, sulfadoxine, sulfaethidole, sulfaguanidine, sulfaguanol, sulfalene, sulfaloxic acid, sulfamerazine, sulfameter, sulfamethazine, sulfamethizole, sulfamethomidine, sulfamethoxazole, sulfamethoxypyridazine, sulfametrole, sulfamidochrysoidine, sulfamoxole, sulfanilamide, 4-sulfanilamidosalicylic acid, n⁴-sulfanilylsuifanilamide, sulfanilylurea, n-sulfanilyl-3,4-xylamide, sulfanitran, sulfaperine, sulfaphenazole, sulfaproxyline, sulfapyrazine, sulfapyridine, sulfasomizole, sulfasymazine, sulfathiazole, sulfathiourea, sulfatolamide, sulfisomidine, sulfisoxazole) sulfones (e.g., acedapsone, acediasulfone, acetosulfone sodium, dapsone, diathymosulfone, glucosulfone sodium, solasulfone, succisulfone, sulfanilic acid, p-sulfanilylbenzylamine, sulfoxone sodium, thiazolsulfone), and others (e.g., clofoctol, hexedine, methenamine, methenamine anhydromethylene citrate, methenamine hippurate, methenamine mandelate, methenamine sulfosalicylate, nitroxoline, taurolidine, xibomol).

Non-limiting examples of immunosuppressive agents include dexamethasone, cyclosporin A, azathioprine, brequinar, gusperimus, 6-mercaptopurine, mizoribine, rapamycin, tacrolimus (FK-506), folic acid analogs (e.g., denopterin, edatrexate, methotrexate, piritrexim, pteropterin, Tomudex®, trimetrexate), purine analogs (e.g., cladribine, fludarabine, 6-mercaptopurine, thiamiprine, thiaguanine), pyrimidine analogs (e.g., ancitabine, azacitidine, 6-azauridine, carmofur, cytarabine, doxifluridine, emitefur, enocitabine, floxuridine, fluorouracil, gemcitabine, tegafur), fluocinolone, triaminolone, anecortave acetate, fluorometholone, medrysone, and prednisolone.

Non-limiting examples of antifungal agents include polyenes (e.g., amphotericin B, candicidin, dermostatin, filipin, fungichromin, hachimycin, hamycin, lucensomycin, mepartricin, natamycin, nystatin, pecilocin, perimycin), azaserine, griseofulvin, oligomycins, neomycin undecylenate, pyirolnitrin, siccanin, tubercidin, viridin, allylamines (e.g., butenafine, naftifine, terbinafine), imidazoles (e.g., bifonazole, butoconazole, chlordantoin, chlormidazole, cloconazole, clotrimazole, econazole, enilconazole, fenticonazole, flutrimazole, isoconazole, ketoconazole, lanoconazole, miconazole, omoconazole, oxiconazole nitrate, sertaconazole, sulconazole, tioconazole), thiocarbamates (e.g., tolciclate, tolindate, tolnaftate), triazoles (e.g., fluconazole, itraconazole, saperconazole, terconazole), acrisorcin, amorolfine, biphenamine, bromosalicylchloranilide, buclosamide, calcium propionate, chlorphenesin, ciclopirox, cloxyquin, coparaffinate, diamthazole dihydrochloride, exalamide, flucytosine, halethazole, hexetidine, loflucarban, nifuratel, potassium iodide, propionic acid, pyrithione, salicylanilide, sodium propionate, sulbentine, tenonitrozole, triacetin, ujothion, undecylenic acid, and zinc propionate.

Non-limiting examples of antiviral agents include acyclovir, carbovir, famciclovir, ganciclovir, penciclovir, and zidovudine.

Non-limiting examples of antiprotozoal agents include pentamidine isethionate, quinine, chloroquine, and mefloquine.

An ophthalmic solution of the present invention is preferably formulated in a physiologically acceptable buffer to regulate pH and tonicity in a range compatible with ophthalmic uses and with any active ingredients present therein. Non-limiting examples of physiologically acceptable buffers include phosphate buffer; a Tris-HCl buffer (comprising tris(hydroxymethyl)aminomethane and HCl); buffers based on HEPES (N-{2-hydroxyethyl}peperazine-N'-{2-ethanesulfonic acid}) having pKₐ of 7.5 at 25 °C and pH in the range of about 6.8-8.2; BES (N,N-bis{2-hydroxyethyl}2-aminoethanesulfonic acid) having pKₐ of 7.1 at 25°C and pH in the range of about 6.4-7.8; MOPS (3-{N-morpholino}propanesulfonic acid) having pKₐ of 7.2 at 25°C and pH in the range of about 6.5-7.9; TES (N-tris{hydroxymethyl}-methyl-2-aminoethanesulfonic acid) having pKₐ of 7.4 at 25°C and pH in the range of about 6.8-8.2; MOBS (4-{N-morpholino}butanesulfonic acid) having pKₐ of 7.6 at 25°C and pH in the range of about 6.9-8.3; DIPSO (3-(N,N-bis{2-hydroxyethyl}amino)-2-hydroxypropane)) having pKₐ of 7.52 at 25°C and pH in the range of about 7-8.2; TAPSO (2-hydroxy-3{tris(hydroxymethyl)methylamino}-1-propanesulfonic acid)) having pKₐ of 7.61 at 25°C and pH in the range of about 7-8.2; TAPS ({(2-hydroxy-1,1-bis(hydroxymethyl)ethyl)amino}-1-propanesulfonic acid)) having pKₐ of 8.4 at 25°C and pH in the range of about 7.7-9.1; TABS (N-tris(hydroxymethyl)methyl-4-aminobutanesulfonic acid) having pKₐ of 8.9 at 25°C and pH in the range of about 8.2-9.6; AMPSO (N-(1,1-dimethyl-2-hydroxyethyl)-3-amino-2-hydroxypropanesulfonic acid)) having pKₐ of 9.0 at 25°C and pH in the range of about 8.3-9.7; CHES (2-cyclohexylamino)ethanesulfonic acid) having pKₐ of 9.5 at 25°C and pH in the range of about 8.6-10.0; CAPSO (3-(cyclohexylamino)-2-hydroxy-1-propanesulfonic acid) having pKₐ of 9.6 at 25°C and pH in the range of about 8.9-10.3; or CAPS (3-(cyclohexylamino)-1-propane sulfonic acid) having pKₐ of 10.4 at 25°C and pH in the range of about 9.7-11.1.

While the buffer itself is a "tonicity adjusting agent" and a "pH adjusting agent" that broadly maintains the ophthalmic solution at a particular ion concentration and pH, additional "tonicity adjusting agents" can be added to adjust or "fine tune" the final tonicity of the solution. Such tonicity adjusting agents are well known to those of skill in the art and include, but are not limited to, mannitol, sorbitol, dextrose, sucrose, urea, and glycerin. Also, various salts, including halide salts of a monovalent cation (e.g., NaCl or KCI) can be utilized.

The tonicity adjusting agent, when present, is preferably in a concentration ranging from about 0.01 to about 10, or from about 0.01 to about 7, or from about 0.01 to about 5, or from about 0.1 to about 2, or from about 0.1 to about 1 percent by weight. In some embodiments where a tonicity adjusting agent is present the solution can contain a single agent or a combination of different tonicity adjusting agents.

Ophthalmic solutions of the present invention also can comprise one or more surfactants. Suitable surfactants can include cationic, anionic, non-ionic or amphoteric surfactants. Preferred surfactants are neutral or nonionic surfactants. Non-limiting examples of surfactants suitable for a formulation of the present invention include polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween® 80, Tween® 60, Tween® 20), poloxamers (synthetic block polymers of ethylene oxide and propylene oxide, such as those commonly known by their trade names of Pluronic®; e.g., Pluronic® F127 or Pluronic® F108)), or poloxamines (synthetic block polymers of ethylene oxide and propylene oxide attached to ethylene diamine, such as those commonly known by their trade names of Tetronic®; e.g., Tetronic® 1508 or Tetronic® 908, etc., other nonionic surfactants such as Brij®, Myrj®, and long chain fatty alcohols (i.e., oleyl alcohol, stearyl alcohol, myristyl alcohol, docosohexanoyl alcohol, etc.) with carbon chains having about 12 or more carbon atoms (e.g., such as from about 12 to about 24 carbon atoms). Such compounds are delineated in Martindale, 34th ed., pp 1411-1416 (Martindale, "The Complete Drug Reference," S. C. Sweetman (Ed.), Pharmaceutical Press, London, 2005) and in Remington, "The Science and Practice of Pharmacy," 21st Ed., pp 291 and the contents of chapter 22, Lippincott Williams & Wilkins, New York, 2006); the contents of these sections are incorporated herein by reference. The concentration of a non-ionic surfactant, when present, in a composition of the present invention can be in the range from about 0.001 to about 5 weight percent (or alternatively, from about 0.01 to about 4, or from about 0.01 to about 2, or from about 0.01 to about 1 weight percent).

In some embodiments, the ophthalmic solutions of this invention can optionally include viscosity adjusting agents (e.g., particularly when the ophthalmic solution is intended to act as a lubricant (i.e., artificial tear)). Suitable viscosity adjusting agents for administration to an eye are well known to those of skill in the art. One or more polyanionic materials disclosed above (especially the polysaccharide-based polyanionic materials) can act as viscosity adjusting agents. However, other polysaccharides (such as the non-ionic polysaccharides) such as cellulose derivatives are commonly used to increase viscosity, and as such, can offer other advantages. Specific cellulose derivatives include, but are not limited to hydroxypropyl methyl cellulose, carboxymethyl cellulose, methyl cellulose, or hydroxyethyl cellulose. Typically, particularly when used as an artificial tear, the ophthalmic solution has a viscosity from about 1 to about 50 centipoises (or Pa.s). As a solution, the present pharmaceutical formulation is usually dispensed in the eye in the form of an eye drop. It should be understood, however, that the present pharmaceutical formulation may also be formulated as a viscous liquid (e.g., viscosities from 50 to several thousand cps), gel, or ointment for other non-ophthalmic uses. Furthermore, in some contact-lens related embodiments, lenses may be soaked or otherwise exposed to a pharmaceutical formulation of the present invention prior to wear.

In some embodiments, an ophthalmic formulation of the present invention can further comprise a demulcent. Polysaccharides, such as those disclosed herein above can act as demulcents. Other demulcents also can be included, such as those approved by the U.S. Food and Drug Administration ("US FDA") and listed in 21 C.F.R. Part 349. They include hypromellose (0.2 to 2.5 percent), dextran 70 (0.1 percent when used with another polymeric demulcent listed in this regulation), gelatin (0.01 percent), liquid polyols, glycerin (0.2 to 1 percent), polyethylene glycol 300 or 400 (0.2 to 1 percent), propylene glycol (0.2 to 1 percent), polyvinyl alcohol (0.1 to 4 percent), povidone (or polyvinyl pyrrolidone, 0.1 to 2 percent). All compositions are in percent by weight of the total formulation.

In some other embodiments, a pharmaceutical formulation may include one or more emollients, such as those listed in 21 C.F.R. Section 349.14.

In addition to those classes of ingredients disclosed above, a pharmaceutical formulation, such as an ophthalmic solution, of the present invention can further comprise one or more other ingredients, such as antioxidants, vitamins, or other ingredients that provide added health benefits to the users. Where an ophthalmic solution is intended for contact-lens care, it can comprise other known components that are generally used for cleaning and maintenance of contact lenses, as long as these components are compatible with other ingredients in the solution. In one embodiment, a contact-lens care solution can comprise microabrasives (e.g., polymer microbeads).

In another embodiment, a pharmaceutical formulation of the present invention can further comprise a second preservative other than a cationic organic nitrogen-containing compound.

In another aspect, the present invention provides a method for preparing a pharmaceutical formulation that comprises at least a polyanionic material and at least a zinc-based compound. The method comprises adding said at least a polyanionic material and at least a zinc-based compound to a formulation.

### Formulation Compounding Procedure

A pharmaceutical formulation of the present invention can be prepared by a method comprising the step of: (a) adding a soluble zinc compound into a vessel containing 80-90 percent of a desired volume of purified water; (b) adding other desired ingredients, such as therapeutic, nutritional, or prophylactic ingredients, which target a desired physiological condition, to form a first mixture; (c) adding at least a polyanionic material to the first mixture to form a second mixture; (d) adding purified water to the vessel to bring the total volume of the second mixture to 100 percent of the desired volume; and (e) mixing the contents of the vessels to produce the pharmaceutical formulation. The method can further comprise subjecting the pharmaceutical formulation to sterilization by heating and/or filtration through a desired filter. Optionally, the method also can comprise adding one or more additional ingredients to the second mixture, which additional ingredients are selected from the group consisting of buffers, tonicity adjusting agents, surfactants, demulcents, emollients, antioxidants, viscosity adjusting agents, vitamins, other ingredients that provide added health benefits to the users, and mixtures thereof.

### Procedure for evaluating the preservative efficacy ("PE") of a pharmaceutical formulation of the present invention against microorganisms

The microorganisms against which the PE of a pharmaceutical formulation of the present invention were *S. aureus* (ATCC 6538), *E. coli* (ATCC 8739), *P*. *aeruginosa* (ATCC 9027), *C. albicans* (ATCC 10231), and *A. niger* (ATCC 16404). This procedure applies to the US FDA premarket notification (510(k)) guidance document and ISO/DIS 14730 standard preservative efficacy testing with a 14-day rechallenge. The evaluations were conducted with 3 separate lots of each test solution for each microorganism. Each lot was tested with a different preparation of each microorganism.

Bacterial cells were grown on Tryptic Soy Agar ("TSA") slants at a temperature in the range from 30 to 35°C in an incubator for a time period from 18 to 24 hours. Fungal cells were grown on Sabouraud Dextrose Agar ("SDA") slants at a temperature in the range from 20°C to 25°C in an incubator for a time period of 2 to 7 days. Cells were harvested in saline solution (5-10 ml, USP, 0.9% saline, with or without 0.1 % Tween 80 surfactant, which was added to each agar slant, followed by gentle agitation with a sterile cotton swab. The cell suspensions were aseptically dispensed into separate sterile polypropylene centrifuge tubes. Cells were harvested by centrifugation at 3000 rpm for 10 minutes, washed one time, and suspended in Saline TS to a concentration of 2 x 10⁸ cells per ml.

The cell suspension (0.1 ml) was diluted with 20 ml of the test solution to reach a final concentration of from 1.0 x 10⁵ to 1.0 x 10⁶ colony-forming units ("CFU"). Phosphate Buffered Saline ("PBS") was used as a control solution. The inoculated test and control solutions were incubated at a temperature ranging from 20°C to 25°C in static culture. At time zero, 1 ml of PBS (USP, pH 7.2) from the control solution was diluted with 9 ml of PBS and serially diluted cells were plated in triplicate on TSA for bacteria and SDA for fungi. The bacterial plates were incubated at a temperature ranging from 30 to 35°C for a period ranging from 2 to 4 days. Fungal plates were incubated at a temperature ranging from 20 to 25°C for a period ranging from 2 to 7 days.

Similarly, at days 7 and 14, a one-milliliter volume from a test solution was added into 9 ml of Dey-Engley neutralizing broth ("DEB") and serially diluted in DEB and plated in triplicate on TSA for bacteria and SDA for fungi. The bacterial plates were incubated at a temperature ranging from 30 to 35°C for a period ranging from 2 to 4 days. Fungal plates were incubated at a temperature ranging from 20°C to 25°C for a period ranging from 2 to 7 days. Developing colonies were counted.

Immediately following the day 14 sampling, test solutions were re-inoculated to give final concentrations of from 1.0 x 10⁴ to 1.0 x 10⁵ of each microorganism. At time zero, 1 ml from the inoculum control was added to 9 ml of PBS and subsequent serial dilutions were plated in triplicate on TSA for bacteria and SDA for fungi. The bacterial plates were incubated at a temperature ranging from 30 to 35°C for a period ranging from 2 to 4 days. Fungal plates were incubated at a temperature ranging from 20 to 25°C for a period ranging from 2 to 7 days.

At days 21 and 28, 1 ml from the test articles was added to 9 ml of DEB and again, serial dilutions were plated in triplicate on TSA. Plates were incubated at a temperature ranging from 30 to 35°C for a period ranging from 2 days to 4 days and developing colonies counted.

Based on the acceptance criteria for bacteria, a solution is acceptable if the concentration of viable bacteria, recovered per milliliter, is reduced by at least 3 logs at day 14, and after a rechallenge at day 14, the concentration of bacteria is reduced by at least 3 logs by day 28. In addition, the solution is acceptable if the concentration of viable yeasts and molds, recovered per milliliter of the solution, remains at or below the initial concentration (within an experimental uncertainty of ± 0.5 log) at day 14, and after a rechallenge at day 14, the concentration of viable yeasts and molds remains at or below the initial concentration (within an experimental uncertainty of ± 0.5 log) at day 28.

The results at the fourteenth and twenty-eighth days for the tested solutions are shown in the following tables as log reduction in the concentration of the applicable microorganism.

### EXAMPLE 1: First formulation

The first formulation had the following composition.

| Ingredient | % W / W |
|---|---|
| Sodium Borate | 0.06 |
| Boric Acid | 0.7 |
| Glycerin | 1 |
| Sodium Alginate | 0.25 |
| Zinc Chloride | 0.025 |
| PE | Failed |

### PE Test Result

| | | |
|---|---|---|
| *S. aureus* | 14 days | > 4.8 |
| | 28 days | > 3.8 |
| *P. aeruginosa* | 14 days | > 4.8 |
| | 28 days | > 3.9 |
| *E. coli* | 14 days | > 4.8 |
| | 28 days | > 3.9 |
| *C. albicans* | 14 days | too numerous to count |
| | 28 days | too numerous to count |
| *A. niger* | 14 days | 1.6 |
| | 28 days | 0.9 |

### EXAMPLE 2: Third formulation

The third formulation had the following composition.

| Ingredient | % W / W |
|---|---|
| Sodium Borate | 0.06 |
| Boric Acid | 0.07 |
| Glycerin | 1 |
| Sodium Alginate | 0.25 |
| Zinc Chloride | 0.025 |
| Magnesium Chloride | 0.01 |
| PE | Passed |

### PE Test Result

| | | |
|---|---|---|
| *S. aureus* | 14 days | > 4.9 |
| | 28 days | > 3.7 |
| *P. aeruginosa* | 14 days | 3.8 |
| | 28 days | > 3.7 |
| *E. coli* | 14 days | 4.8 |
| | 28 days | > 3.7 |
| *C. albicans* | 14 days | 0.7 |
| | 28 days | 1.0 |
| *A. niger* | 14 days | 1.3 |
| | 28 days | 1.1 |

### EXAMPLE 3 Seventh formulation

The seventh formulation had the following composition.

| Ingredient | % W / W |
|---|---|
| Sodium Borate | 0.115 |
| Boric Acid | 0.5 |
| Glycerin | 1 |
| Sodium Alginate | 0.25 |
| Zinc Chloride | 0.01 |
| Magnesium Chloride | 0.01 |
| PE | Passed |

### PE Test Result

| | | |
|---|---|---|
| *S. aureus* | 14 days | > 4.8 |
| | 28 days | > 3.8 |
| *P. aeruginosa* | 14 days | > 4.8 |
| | 28 days | > 3.9 |
| *E. coli* | 14 days | > 4.8 |
| | 28 days | > 3.9 |
| *C. albicans* | 14 days | 0.4 |
| | 28 days | 1.8 |
| *A. niger* | 14 days | 1.4 |
| | 28 days | -0.1 |

### EXAMPLE 4 Tenth formulation

The tenth formulation had the following composition.

| Ingredient | % W/W |
|---|---|
| Sodium Borate | 0.014 |
| Boric Acid | 0.5 |
| Glycerin | 1 |
| Sodium Hyaluronate | 0.25 |
| Zinc Chloride | 0.02 |
| Magnesium Chloride | 0.01 |
| PE | Passed |

### PE Test Result

| | | |
|---|---|---|
| *S. aureus* | 14 days | > 4.7 |
| | 28 days | > 4.7 |
| *P. aeruginosa* | 14 days | > 4.7 |
| | 28 days | 4.7 |
| *E. coli* | 14 days | > 4.8 |
| | 28 days | > 4.8 |
| C. *albicans* | 14 days | 0.9 |
| | 28 days | 1.5 |
| *A. niger* | 14 days | 1.3 |
| | 28 days | 1.4 |

### EXAMPLE 5 Eleventh formulation

The eleventh formulation had the following composition.

| Ingredient | % W / W |
|---|---|
| Sodium Borate | 0.014 |
| Boric Acid | 0.5 |
| Glycerin | 1 |
| Sodium Hyaluronate | 0.25 |
| Zinc Chloride | 0.02 |
| Magnesium Chloride | 0.02 |
| PE | Passed |

### PE Test Result

| | | |
|---|---|---|
| *S. aureus* | 14 days | > 4.7 |
| | 28 days | > 4.7 |
| *P. aeruginosa* | 14 days | > 4.7 |
| | 28 days | > 4.7 |
| *E. coli* | 14 days | > 4.8 |
| | 28 days | > 4.8 |
| C. *albicans* | 14 days | 0.8 |
| | 28 days | 1.6 |
| *A. niger* | 14 days | 1.4 |
| | 28 days | 1.4 |

### EXAMPLE 6 Twelfth formulation

The twelfth formulation had the following composition.

| Ingredient | % W / W |
|---|---|
| Sodium Borate | 0.014 |
| Boric Acid | 0.5 |
| Glycerin | 1 |
| Sodium Hyaluronate | 0.25 |
| Zinc Chloride | 0.025 |
| Magnesium Chloride | 0.01 |
| PE | Passed |

### PE Test Result

| | | |
|---|---|---|
| *S. aureus* | 14 days | > 4.7 |
| | 28 days | > 4.7 |
| *P. aeruginosa* | 14 days | > 4.7 |
| | 28 days | > 4.7 |
| *E. coli* | 14 days | > 4.8 |
| | 28 days | > 4.8 |
| *C. albicans* | 14 days | 0.9 |
| | 28 days | 1.6 |
| *A. niger* | 14 days | 1.8 |
| | 28 days | 1.3 |

### EXAMPLE 7 Thirteenth formulation

The thirteenth formulation had the following composition.

| Ingredient | % W / W |
|---|---|
| Sodium Borate | 0.014 |
| Boric Acid | 0.5 |
| Glycerin | 1 |
| Sodium Hyaluronate | 0.5 |
| Zinc Chloride | 0.02 |
| Magnesium Chloride | 0.01 |
| PE | Passed |

### PE Test Result

| | | |
|---|---|---|
| *S*. *aureus* | 14 days | > 4.7 |
| | 28 days | > 4.7 |
| *P. aeruginosa* | 14 days | 3.4 |
| | 28 days | > 4.7 |
| *E. coli* | 14 days | > 4.8 |
| | 28 days | > 4.8 |
| *C. albicans* | 14 days | 1 |
| | 28 days | 1.7 |
| *A. niger* | 14 days | 1.3 |
| | 28 days | 1.3 |

### EXAMPLE 8 Fourteenth formulation

The fourteenth formulation had the following composition.

| Ingredient | % W / W |
|---|---|
| Sodium Borate | 0.014 |
| Boric Acid | 0.5 |
| Glycerin | 1 |
| Sodium Hyaluronate | 0.5 |
| Zinc Chloride | 0.02 |
| Magnesium Chloride | 0.02 |
| PE | Passed |

### PE Test Result

| | | |
|---|---|---|
| *S. aureus* | 14 days | > 4.7 |
| | 28 days | > 4.7 |
| *P. aeruginosa* | 14 days | > 4.7 |
| | 28 days | > 4.7 |
| *E. coli* | 14 days | > 4.8 |
| | 28 days | > 4.8 |
| *C. albicans* | 14 days | 1 |
| | 28 days | 1.7 |
| *A. niger* | 14 days | 1.8 |
| | 28 days | 1.4 |

### EXAMPLE 9 Fifteenth formulation

The fifteenth formulation had the following composition.

| Ingredient | % W / W |
|---|---|
| Sodium Borate | 0.014 |
| Boric Acid | 0.5 |
| Glycerin | 1 |
| Sodium Hyaluronate | 0.25 |
| Zinc Chloride | 0.01 |
| PE | Passed |

### PE Test Result

| | | |
|---|---|---|
| *S. aureus* | 14 days | > 4.7 |
| | 28 days | > 4.7 |
| *P. aeruginosa* | 14 days | > 4.7 |
| | 28 days | > 4.7 |
| *E. coli* | 14 days | > 4.8 |
| | 28 days | > 4.8 |
| *C. albicans* | 14 days | 0.7 |
| | 28 days | 1.6 |
| *A. Niger* | 14 days | 1.9 |
| | 28 days | 1.5 |

### EXAMPLE 18 Sixteenth formulation

The sixteenth formulation had the following composition.

| Ingredient | % W / W |
|---|---|
| Sodium Borate | 0.014 |
| Boric Acid | 0.5 |
| Glycerin | 1 |
| Sodium Hyaluronate | 0.25 |
| Zinc Chloride | 0.02 |
| PE | Passed |

### PE Test Result

| | | |
|---|---|---|
| *S. aureus* | 14 days | > 4.7 |
| | 28 days | > 4.7 |
| *P. aeruginosa* | 14 days | > 4_{.}7 |
| | 28 days | > 4.7 |
| *E. coli* | 14 days | > 4.8 |
| | 28 days | > 4.7 |
| *C. albicans* | 14 days | 0.9 |
| | 28 days | 1.6 |
| *A. niger* | 14 days | 1.8 |
| | 28 days | 1.3 |

### EXAMPLE 11 Seventeenth formulation

The seventeenth formulation had the following composition.

| Ingredient | % W / W |
|---|---|
| Sodium Borate | 0.014 |
| Boric Acid | 0.5 |
| Glycerin | 1 |
| Sodium Hyaluronate | 0.25 |
| Zinc Chloride | 0.03 |
| PE | Passed |

### PE Test Result

| | | |
|---|---|---|
| *S*. *aureus* | 14 days | > 4.7 |
| | 28 days | 4.7 |
| *P. aeruginosa* | 14 days | > 4.7 |
| | 28 days | > 4.7 |
| *E. coli* | 14 days | > 4.8 |
| | 28 days | > 4.7 |
| *C. albicans* | 14 days | 1 |
| | 28 days | 1.8 |
| *A. niger* | 14 days | 1.8 |
| | 28 days | 1.4 |

In another aspect, an ophthalmic solution of the present invention can be used to treat ocular conditions such as dry eye, inflammation, or infection of the eye.

In still another aspect, the present invention provides methods of making and using a pharmaceutical formulation of the present invention. Any of the materials, compounds, and ingredients disclosed herein is applicable for use with or inclusion in any method of the present invention.

In still another aspect, the present invention provides a method for making a pharmaceutical formulation. The method comprises providing at least a polyanionic material and at least a zinc-based compound in the pharmaceutical formulation. In one embodiment, the method comprises: (a) providing an initial formulation; and (b) adding said at least a polyanionic material and said at least a zinc-based compound to the initial formulation to produce the pharmaceutical formulation. In another embodiment, the method further comprises adding another ingredient selected from the group consisting of therapeutic agents, buffers, tonicity adjusting agents, surfactants, viscosity adjusting agents, and other agents to the pharmaceutical formulation. The therapeutic agents can be selected from the group of anti-inflammatory agents, antibiotics, immunosuppressive agents, antiviral agents, antifungal agents, and antiprotozoal agents. In still another embodiment, the zinc-based compound comprises a compound that is soluble In an aqueous medium. Non-limiting examples of each of these classes of agents, compounds, and ingredients are throughout the present specification.

In still another aspect, the present invention provides a method for providing safety, or comfort, or both to users of a pharmaceutical formulation. The method comprises adding at least a polyanionic material and at least a zinc-based compound to the pharmaceutical formulation. The zinc-based compound is zinc chloride the polyanionic material is selected from physiologically acceptable salts of alginic acid, and hyaluronic acid;

In yet another aspect, the present invention provides a method for treating or preventing a condition of an eye that manifests irritation or inflammation. The method comprises topically administering to the eye an effective amount of an ophthalmic solution that comprises at least a polyanionic material and at least a soluble zinc compound to relieve such irritation or inflammation. In one embodiment, the method is used for treating a dry eye condition. In another embodiment, the method for treating or relieving symptoms of dry eye comprises administering to an ocular surface an effective amount of an ophthalmic solution that comprises a polyanionic material, a soluble zinc compound, a demulcent, a tonicity adjusting agent, and a buffering agent.

In a further aspect, the present invention provides a method for treating an ophthalmic device. The method comprises contacting the ophthalmic device with an ophthalmic solution comprising at least a polyanionic material and at least a soluble zinc compound. In one embodiment, the ophthalmic solution has the capability to clean, disinfect, and wet or rewet the ophthalmic device. The ophthalmic solution comprises a polyanionic material, a soluble zinc compound, a surfactant, and a tonicity adjusting agent. The ophthalmic solution can further comprise a buffering agent.

In still a further aspect, the ophthalmic device is a contact lens.

In a further aspect, the present invention provides a use of at least a polyanionic material and at least a zinc-based compound for the preparation of a pharmaceutical formulation, such as an ophthalmic solution. In some embodiments of the present invention, the preparation can further include the use of additional ingredients, such as therapeutic agents, buffers, tonicity adjusting agents, surfactants, viscosity adjusting agents, other agents, combinations thereof, or mixtures thereof.

In yet another aspect, the zinc-based compound is included in a formulation in an amount sufficient to reduce the concentration of bacteria by at least 3 logs reduction at the fourteenth day after challenge with said bacteria, and to reduce the concentration of bacteria by at least 3 logs reduction at the twenty-eighth day after rechallenge with said bacteria at the fourteenth day. In addition, in further embodiments, the amount of the zinc-based compound is also sufficient to keep the concentration of yeasts and molds at the fourteenth day after challenge with said yeasts and molds at or below the initial concentration, and to keep the concentration of yeasts and molds at the twenty-eighth day after rechallenge with said yeasts and molds at the fourteenth day at or below the initial concentration.

## Claims

1. An ophthalmic pharmaceutical formulation consisting of purified water and:
(a) 0.06 wt.-% sodium borate; 0.7 wt.-% boric acid; 1wt.-% glycerin, 0.25 wt.-% sodium alginate; 0.025 wt.-% zinc chloride; and optionally 0.01 wt.-% magnesium chloride; or
(b) 0.115 wt.-% sodium borate; 0.5 wt.-% boric acid; 1 wt.-% glycerin, 0.25 wt.-% sodium alginate; 0.01 wt.-% zinc chloride; and 0.01 wt.-% magnesium chloride; or
(c) 0.014 wt.-% sodium borate; 0.5 wt.-% boric acid; 1 wt.-% glycerin, 0.25 wt.-% or 0.5 wt.-% sodium hyaluronate; 0.02 wt.-% zinc chloride; and 0.01 wt.-% magnesium chloride; or
(d) 0.014 wt.-% sodium borate; 0.5 wt.-% boric acid; 1 wt.-% glycerin, 0.25 wt.-% or 0.5 wt.-% sodium hyaluronate; 0.02 wt.-% zinc chloride; and 0.02 wt.-% magnesium chloride; or
(e) 0.014 wt.-% sodium borate; 0.5 wt.-% boric acid; 1 wt.-% glycerin, 0.25 wt.-% sodium hyaluronate; 0.025 wt.-% zinc chloride; and 0.01 wt.-% magnesium chloride; or
(f) 0.014 wt.-% sodium borate; 0.5 wt.-% boric acid; 1 wt.-% glycerin, 0.25 wt.-% sodium hyaluronate; and 0.01 wt.-% or 0.02 wt.-% or 0.03 wt.-% zinc chloride;
wherein all wt.-% are based on the total weight of the formulation.

2. The ophthalmic pharmaceutical formulation of claim 1, wherein the pharmaceutical formulation comprises a contact-lens care solution.

3. The ophthalmic pharmaceutical formulation of claim 1 for use in providing ocular safety, or comfort, or both to users of said ophthalmic pharmaceutical formulation.

4. An ophthalmic solution for use in treating of or preventing of a condition of an eye that manifests irritation or inflammation, the solution comprising an effective amount of the ophthalmic pharmaceutical formulation of claim 1.

5. A method for treating an ophthalmic device, the method comprising contacting the ophthalmic device with the ophthalmic pharmaceutical formulation of claim 1.

## Patentansprüche

1. Eine ophthalmische pharmazeutische Formulierung bestehend aus gereinigtem Wasser und:
(a) 0.06 Gew.-% Natriumborat; 0.7 Gew.-% Borsäure; 1 Gew.-% Glycerin, 0.25 Gew.-% Natriumalginat; 0.025 Gew.-% Zinkchlorid; und wahlweise 0.01 Gew.-% Magnesiumchlorid; oder
(b) 0.115 Gew.-% Natriumborat; 0.5 Gew.-% Borsäure; 1 Gew.-% Glycerin, 0.25 Gew.-% Natriumalginat; 0.01 Gew.-% Zinkchlorid; und 0.01 Gew.-% Magnesiumchlorid; oder
(c) 0.014 Gew.-% Natriumborat; 0.5 Gew.-% Borsäure; 1 Gew.-% Glycerin, 0.25 Gew.-% oder 0.5 Gew.-% Natriumhyaluronat; 0.02 Gew.-% Zinkchlorid; und 0.01 Gew.-% Magnesiumchlorid; oder
(d) 0.014 Gew.-% Natriumborat; 0.5 Gew.-% Borsäure; 1 Gew.-% Glycerin, 0.25 Gew.-% oder 0.5 Gew.-% Natriumhyaluronat; 0.02 Gew.-% Zinkchlorid; und 0.02 Gew.-% Magnesiumchlorid; oder
(e) 0.014 Gew.-% Natriumborat; 0.5 Gew.-% Borsäure; 1 Gew.-% Glycerin, 0.25 Gew.-% Natriumhyaluronat; 0.025 Gew.-% Zinkchlorid; und 0.01 Gew.-% Magnesiumchlorid; oder
(f) 0.014 Gew.-% Natriumborat; 0.5 Gew.-% Borsäure; 1 Gew.-% Glycerin, 0.25 Gew.-% Natriumhyaluronat; und 0.01 Gew.-% oder 0.02 Gew.-% oder 0.03 Gew.-% Zinkchlorid;
wobei alle Gew.-%-Angaben auf das Gesamtgewicht der Formulierung bezogen sind.

2. Die ophthalmische pharmazeutische Formulierung aus Anspruch 1, wobei die pharmazeutische Formulierung eine Kontaktlinsenpflegelösung umfasst.

3. Die ophthalmische pharmazeutische Formulierung aus Anspruch 1 zur Verwendung in der Bereitstellung von Augensicherheit oder -komfort oder beidem bei Benutzern der besagten ophthalmischen pharmazeutischen Formulierung.

4. Eine ophthalmische Lösung zur Verwendung in der Behandlung oder zur Vorbeugung von sich manifestierenden Reizungs- oder Entzündungszuständen des Auges, wobei die Lösung eine wirksame Menge der ophthalmischen pharmazeutischen Formulierung aus Anspruch 1 umfasst.

5. Eine Methode zur Behandlung einer ophthalmischen Vorrichtung, wobei die Methode das Inkontaktbringen der ophthalmischen Vorrichtung mit der ophthalmischen pharmazeutischen Formulierung aus Anspruch 1 umfasst.

## Revendications

1. Formulation pharmaceutique à usage ophtalmique constituée d'eau purifiée et de :
(a) 0,06 % en poids de borate de sodium ; 0,7 % en poids d'acide borique ; 1 % en poids de glycérol ; 0,25 % en poids d'alginate de sodium ; 0,025 % en poids de chlorure de zinc ; et éventuellement 0,01 % en poids de chlorure de magnésium ; ou
(b) 0,115 % en poids de borate de sodium ; 0,5 % en poids d'acide borique ; 1 % en poids de glycérol ; 0,25 % en poids d'alginate de sodium ; 0,01 % en poids de chlorure de zinc ; et 0,01 % en poids de chlorure de magnésium ; ou
(c) 0,014 % en poids de borate de sodium ; 0,5 % en poids d'acide borique ; 1 % en poids de glycérol ; 0,25 % en poids ou 0,5 % en poids d'hyaluronate de sodium ; 0,02 % en poids de chlorure de zinc ; et 0,01 % en poids de chlorure de magnésium ; ou
(d) 0,014 % en poids de borate de sodium ; 0,5 % en poids d'acide borique ; 1 % en poids de glycérol ; 0,25 % en poids ou 0,5 % en poids d'hyaluronate de sodium ; 0,02 % en poids de chlorure de zinc ; et 0,02 % en poids de chlorure de magnésium ; ou
(e) 0,014 % en poids de borate de sodium ; 0,5 % en poids d'acide borique ; 1 % en poids de glycérol ; 0,25 % en poids d'hyaluronate de sodium ; 0,025 % en poids de chlorure de zinc ; et 0,01 % en poids de chlorure de magnésium ; ou
(f) 0,014 % en poids de borate de sodium ; 0,5 % en poids d'acide borique ; 1 % en poids de glycérol ; 0,25 % en poids d'hyaluronate de sodium ; et 0,01 % en poids ou 0,02 % en poids ou 0,03 % en poids de chlorure de zinc ;
dans laquelle tous les pourcentages en poids sont basés sur le poids total de la formulation.

2. Formulation pharmaceutique à usage ophtalmique selon la revendication 1, laquelle formulation pharmaceutique comprend une solution pour l'entretien des lentilles de contact.

3. Formulation pharmaceutique à usage ophtalmique selon la revendication 1, pour utilisation afin d'offrir une sécurité ou un confort oculaire, ou les deux, à des utilisateurs de ladite formulation pharmaceutique à usage ophtalmique.

4. Solution ophtalmique pour utilisation dans le traitement ou la prévention d'un état d'un oeil qui manifeste une irritation ou une inflammation, la composition comprenant une quantité efficace de la formulation pharmaceutique à usage ophtalmique de la revendication 1.

5. Procédé pour traiter un dispositif ophtalmique, le procédé comprenant la mise en contact du dispositif ophtalmique avec la formulation pharmaceutique à usage ophtalmique de la revendication 1.
